# FASCICULE DE BREVET EUROPEEN

(11) **EP 2 309 981 B1**
(45) Date de publication et mention de la délivrance du brevet: **08.05.2013**
(21) Numéro de dépôt: 09730472.9
(22) Date de dépôt: 23.03.2009
(51) Int. Cl.: A61K 9/00, A61K 31/327, A61P 17/00

(54) **COMPOSITION DERMATOLOGIQUE DESTINEE AU TRAITEMENT DES ESCARRES**
DERMATOLOGISCHE ZUSAMMENSETZUNG ZUR BEHANDLUNG VON WUNDSCHORF
DERMATOLOGICAL COMPOSITION FOR THE TREATMENT OF SCABS

(30) Priorité: 03.04.2008 FR 0852225
(43) Date de publication de la demande: 20.04.2011
(73) Titulaire: Laboratoires Carilene, 78360 Montesson (FR)
(72) Inventeur: DESJONQUERES, Stéphane, F-78600 Maisons Laffitte (FR)
(74) Mandataire: Hubert, Philippe
(86) Numéro de dépôt international: PCT/FR2009/050496
(87) Numéro de publication internationale: WO 2009/125117

(56) Documents cités:
- EP-A- 0 117 962
- EP-A- 0 293 535
- WO-A-03/041688
- WO-A-2006/087359
- WO-A-2007/137881
- FR-A- 2 461 744
- US-A1- 2005 266 094
- L. DANN ET AL.: "Experimental wounds treated with cod-liver oil" THE LANCET, 1942, pages 95-98, XP002509664
- C. LULEY ET AL.: "Fettsäuren-Zusammensetzung und Peroxidationsgrad in Fischöl- und Lebertran-Präparaten" ARTZNEIMITTEL-FORSCHUNG, vol. 38, no. 12, 1988, pages 1783-1786, XP008100168
- G. DOEE: "Les indices de peroxide en cosmétologie" COSMETOLOGIE, vol. 3, 1960, pages 14-20, XP008100147
- E. KARDASH-STROCHKOVA ET AL.: "Redox potentiometric determination of peroxide value in edible oils without titration." TALANTA, vol. 54, 2001, pages 411-416, XP002509665

## Description

La présente invention concerne une nouvelle composition formulée pour une application topique sur la peau destinée au traitement des escarres.

On connaît différents lipides peroxydés, notamment obtenus par peroxydation d'huiles végétales naturelles. De tels lipides peroxydés et leurs utilisations ont été décrits dans les brevets suivants BSM N°2 330 M, EP 0 293 535, FR-A-2 591 112, EP-A-225 831, EP-A-225 832, EP-A-225 833, EP-A-226 506, FR-A-2 461 744, FR-A-2 539 142 et EP-A-117 962 qui concernent soit la préparation de tels lipides peroxydés soit leurs applications dans différents domaines, en particulier dans le soin de certaines affections dans le domaine de la rhumatologie ou de la traumatologie, ou encore en tant que produits participant au processus de cicatrisation.

On a décrit notamment dans le brevet européen EP 0 293 535 cité ci-dessus l'utilisation de lipides peroxydés dans la réparation des peaux fragilisées des personnes se trouvant en position allongée pendant des périodes prolongées, dont le stade final est l'escarre et qui constitue une véritable hantise, tant pour le malade qui en souffre, que pour le personnel hospitalier qui cherche à secourir cette souffrance. L'escarre est en effet une nécrose de la peau créée par un manque de vascularisation, aussi bien des capillaires que des veines et des artères, du fait de la compression de la surface cutanée du patient sur la surface sur laquelle il est couché.

L'utilisation d'huile peroxydée pour réparer les peaux fragilisées des personnes se trouvant en positon allongée constitue, comme cela ressort du brevet EP 0 293 535, un moyen particulièrement efficace pour soigner et prévenir les escarres, en réalisant notamment un massage avec ce produit de la zone à traiter.

A l'heure actuelle, il existe une recherche intense pour mettre au point des produits permettant d'améliorer une condition pathologique par la seule intervention d'une action mécanique, sans action pharmacologique.

A titre d'exemple de tels produits, on peut citer des produits formulés de façon à créer, à la manière d'un pansement, une barrière isolante à la surface d'une plaie de façon à permettre la cicatrisation naturelle de cette plaie en l'isolant du milieu extérieur.

Un autre exemple de domaines où l'on recherche des produits agissant sur une condition douloureuse uniquement par un effet mécanique est celui du massage thérapeutique. En effet, il est bien connu qu'un certain nombre de conditions douloureuses ou pathologiques répondent favorablement à l'action d'un massage. Toutefois, le massage est d'autant plus efficace qu'il s'effectue dans un environnement mécanique favorable, favorisant en particulier la glisse des mains du masseur sur la surface du corps où s'applique le massage. On cherche également souvent à améliorer la douceur de ce massage et à éviter toute irritation de la peau lors de massages répétés ou longs. Il est courant d'utiliser à cette fin des produits adjuvants tels que des talcs plus ou moins élaborés destinés à faciliter ce massage.

Dans le cadre de ses recherches sur de nouveaux moyens d'amélioration de conditions pathologiques par la seule intervention d'une action mécanique, l'inventeur de la présente invention a découvert et décrit dans la demande FR 2797586 que les lipides peroxydés pouvaient être utilisés de façon particulièrement efficace en tant qu'agents destinés à former un film lipidique à la surface de la peau et que la formation de ce film lipidique permettait, de façon remarquable, d'améliorer un certain nombre de conditions pathologiques, en particulier en permettant, par un effet protecteur, une amélioration de la cicatrisation des plaies, le traitement de différents érythèmes. Plus généralement, il s'est avéré que le film lipidique formé par des lipides peroxydés était particulièrement efficace dans toutes les applications où l'on cherche à isoler la peau du milieu extérieur pour améliorer ses qualités, en particulier ses qualités biomécaniques.

Il s'est également avéré que les lipides peroxydés pouvaient être utilisés comme adjuvant de massage et cela, en liaison avec la formation d'un film particulièrement efficace formé sur la partie du corps à masser avant ou au cours du massage qui facilite la « glisse » au moment du massage.

Ce type de mode d'action s'avère particulièrement intéressant à une époque où l'on recherche des modes de traitement aussi peu agressifs que possible pour l'organisme. De tels traitements permettent, en outre, de satisfaire à la réglementation de la Communauté Européenne concernant les dispositifs médicaux, puisque ces derniers couvrent dans un chapitre particulier des produits permettant d'escompter des bénéfices sur la santé associés à des produits d'usage local par le seul effet mécanique lié à leur emploi.

L'administration par voie orale de l'huile de foie de morue est, de longue date, bien connue puisque cette huile a été utilisée de longue date pour assurer le bon développement des enfants, notamment en ce qui concerne le squelette, le système nerveux et le développement intellectuel. Cette utilisation par voie orale existe toujours à l'heure actuelle, avec toutefois des formulations, par exemple sous forme de gélules, pour éviter les désagréments liés à l'odeur particulièrement forte de l'huile de foie de morue.

Outre, cette utilisation par voie interne, l'huile de foie de morue a été également décrite pour son intérêt en application topique. On a ainsi pu signaler dans la littérature que l'huile de foie de morue pouvait favoriser la guérison des plaies et la qualité de la peau.

Ainsi, il a été décrit dans « Topical application of cod liver oil ointment accelerates wound healing : an experimental study in wounds in the ears of hairless mice », Scand J Plast Reconstr Hand Surg 34 : 15-20, 2000, l'effet de l'application topique d'huile de foie de morue sur la vitesse d'épithélialisation et de néovascularisation sur un modèle de souris sans poils ainsi que l'effet de l'application locale de vitamine A sur le même modèle.

Cette étude a confirmé l'effet cicatrisant de l'huile de foie de morue et cherché à expliquer cette activité qui semble être liée à la présence de quantités importantes de vitamine A dans l'huile de foie de morue.

Il est, en effet, bien connu que la vitamine A est utilisée dans de nombreuses applications cosmétiques pour son activité sur la régularisation de la kératinisation de la peau et sur les modifications qu'elle permet d'apporter au derme, en agissant sur le métabolisme des fibroblastes.

Toutefois, cette utilisation envisagée de l'huile de foie de morue, a sans doute été largement minimisée du fait de l'odeur désagréable du produit et, cela malgré les traitements que l'on peut faire subir à cette huile pour diminuer l'odeur désagréable.

Poursuivant ses études en vue d'améliorer la prévention et le soin des escarres mais également d'améliorer le résultat et les conditions du massage de la peau ainsi que le soin des peaux irritées et/ou présentant des rougeurs et/ou présentant des lésions superficielles, tout en évitant le recours à des compositions induisant un effet pharmacologique, métabolique ou immunologique, l'inventeur a maintenant découvert qu'il était possible d'améliorer encore l'efficacité des huiles peroxydées telle qu'elle a été décrite à ce jour en les combinant à de l'huile de foie de morue, en particulier dans des proportions telles que l'on bénéficie des avantages des deux types de produits, en évitant en outre l'inconvénient bien connu de l'huile de foie de morue qui est son odeur, du fait notamment de la véritable "dilution" de l'huile de foie de morue dans l'huile peroxydée.

L'inventeur de la présente invention a en effet pu constater qu'il était possible d'améliorer grandement les résultats obtenus par application topique de compositions à base d'huiles végétales peroxydées, notamment dans des opérations de massage, et tout particulièrement dans le traitement des escarres, en mélangeant ces huiles à de l'huile de foie de morue. La composition ainsi obtenue présente les avantages des deux types d'huiles tout en minimisant les inconvénients liés à l'utilisation de l'huile de foie de morue et tout particulièrement à son odeur.

Il est ainsi apparu que les deux types d'huiles pouvaient agir en synergie étant parfaitement miscibles l'un dans l'autre, du fait des propriétés rhéologiques très voisines des deux types d'huiles.

Il a ainsi été possible de préparer des compositions huileuses plus ou moins visqueuses, en particulier des gels, ou des gels mixtes ou des émulsions qui présentent à la fois les avantages des compositions à base d'huiles peroxydées décrites dans la littérature citée ci-dessus en y associant ceux de l'huile de foie de morue dont un des avantages, outre ses propriétés cicatrisantes sur la peau, est d'apporter à la peau des acides gras essentiels que l'on ne trouve pas en quantité suffisante dans les huiles végétales peroxydées, en particulier des acides oméga 3 ou acide alpha linolénique et oméga 6 ou acide linolénique. Ces deux types d'acides gras appelés acides gras essentiels permettent à l'organisme de synthétiser d'autres acides gras indispensables.

Un avantage tout particulier des compositions de l'invention est qu'elles n'induisent aucun effet pharmacologique, métabolique ou immunologique, puisqu'elles ne contiennent pas de composés pharmacologiques et sont constituées essentiellement de lipides peroxydés (triesters de glycérol oxydés) et d'huile de foie de morue, les autres constituants étant pour l'essentiel éventuellement de l'eau, des arômes ou des parfums, des conservateurs ou des agents destinés à régler la viscosité de la composition pour améliorer l'application topique sur la peau.

Un avantage particulier de ce type de compositions et du mode d'action mis en oeuvre lors de son application (formation d'un film lipidique sur la zone à traiter) est qu'elles s'avèrent particulièrement intéressantes à une époque où l'on recherche tout particulièrement des modes de traitement aussi peu agressifs que possible pour l'organisme. Ces compositions permettent en outre de satisfaire à la réglementation de la Communauté Européenne sur les dispositifs médicaux et à celle de la Food and Drug Administration aux USA, concernant les produits de type « monographe » (monograph products) ou dispositifs médicaux (médical devices).

Ainsi, selon un premier aspect, l'invention concerne à titre de produit industriel nouveau, une composition formulée pour une application topique sur la peau, caractérisée en ce qu'elle contient, à titre de constituants essentiels, une huile peroxydée d'origine végétale ou un mélange d'huiles peroxydées d'origine végétale présentant un taux de peroxydation compris entre 30 et 500 milliéquivalents par kg et de l'huile de foie de morue, pour son utilisation dans le soin et/ou la prévention des escarres.

Selon un deuxième aspect, l'invention concerne un dispositif médical constitué d'une composition faisant l'objet du premier aspect.

Selon un troisième aspect, l'invention concerne l'utilisation d'une association d'huile(s) peroxydée(s) et d'huile de foie de morue pour la préparation d'un dispositif médical faisant l'objet du deuxième aspect, ledit dispositif médical agissant pour former un film protecteur sur la peau, notamment pour le soin des peaux irritées et/ou présentant des rougeurs et/ou présentant des lésions superficielles, et/ou étant utilisé comme adjuvant dans des opérations de massage de la peau.

L'invention concerne également un dispositif médical agissant pour former un film protecteur sur la peau, notamment pour le soin des peaux irritées et/ou présentant des rougeurs et/ou présentant des lésions superficielles et/ou utilisé comme adjuvant dans des opérations de massage de la peau, en particulier pour le soin et/ou la prévention des escarres.

Par "constituants essentiels" au sens de l'invention, il faut comprendre que l'huile végétale peroxydée ou le mélange d'huiles végétales peroxydées et l'huile de foie de morue sont les constituants de base de la composition.

Ces deux constituants du fait de leur compatibilité permettront la formation sur la peau à traiter d'un film lipidique qui agira comme un véritable pansement. A l'abri de ce pansement, la régénération naturelle de la peau est accélérée et ainsi la cicatrisation facilitée.

Par ailleurs, la composition, en particulier lorsqu'elle sera sous forme huileuse ou sous forme d'un gel huileux aura, du fait de la présence des deux types de produits compatibles qui sont des produits huileux un avantage essentiel pour favoriser la « glisse » lors d'une opération de massage.

La composition de l'invention pourra en outre contenir de l'eau, notamment en quantité suffisante pour que la solution se trouve sous forme d'une émulsion de type huile dans eau ou pour former avec l'huile peroxydée ou le mélange d'huiles peroxydées et l'huile de foie de morue un gel de type "gel mixte", en présence d'un agent de viscosification.

Par "gel mixte" au sens de l'invention, on entend un gel à base d'huile et d'eau, l'huile étant constituée dans le cas présent par les huiles peroxydées et l'huile de foie de morue contenues dans la composition de l'invention.

Outre les constituants essentiels, qui sont les constituants actifs, huile(s) peroxydée(s) et l'huile de foie de morue, et éventuellement l'eau, la composition de l'invention pourra renfermer différents additifs tels que notamment des parfums ou des arômes choisis en fonction de l'application de la composition, des agents conservateurs ou des agents destinés à régler la viscosité de la composition.

La composition pourra en outre, contenir différents adjuvants de formulation destinés notamment à améliorer la galénique, à faciliter l'application ou à améliorer la protection de la composition.

De tels adjuvants de formulation seront, de façon classique, des agents émulsionnants, émulsifiants, des tensio-actifs, des agents mouillants, émollients, conservateurs ou antioxydants.

Le degré de peroxydation de l'huile végétale peroxydée ou du mélange d'huiles végétales peroxydées est mesuré selon la norme ISO 3960.

Pour la préparation des compositions de la présente invention, on choisira des huiles ou des mélanges d'huiles végétales peroxydées présentant un taux de peroxydation compris entre 30 et 500 milliéquivalents par kg.

Ce taux de peroxydation sera de façon encore plus avantageuse compris entre 50 et 300 milliéquivalents par kg, de préférence encore entre 50 et 150 milliéquivalents par kg.

A titre d'exemples d'huiles végétales choisie selon l'invention, on citera, l'huile d'amande douce, l'huile de noisette, l'huile d'arachide, l'huile de maïs, l'huile de pépin de raisin, l'huile de sésame et l'huile de carthame. On pourra également utiliser un mélange de ces huiles.

Selon une variante particulièrement préférée de l'invention, on choisira une huile de maïs peroxydée présentant un taux de peroxydation compris entre 30 et 500 milliéquivalents par kg.

Les huiles ou mélanges d'huiles végétales utilisés selon l'invention sont d'une façon avantageuse constitués, à titre de constituants majoritaires, représentant généralement au moins 80 % de la masse de triglycérides répondant à la formule 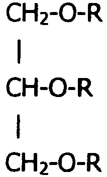 dans laquelle les radicaux R sont majoritairement représentés par des acides insaturés en C18 partiellement peroxydés (en fonction du taux de peroxydation de ladite huile).

La composition de l'invention contiendra avantageusement de 0,5 à 20 %, de préférence de 1 à 10 % en poids d'huile de foie de morue.

Selon une variante avantageuse de la présente invention, on utilise de l'huile de foie de morue ayant subi un traitement préalable destiné à réduire les odeurs et tout particulièrement, un traitement de raffinage à froid, de préférence un traitement de winterisation. De telles huiles de foie de morue sont disponibles dans le commerce.

Les compositions de l'invention sont avantageusement sous la forme d'une émulsion ou d'un gel.

Lorsque la composition est sous forme d'une émulsion, il s'agira avantageusement d'une émulsion de type huile dans eau. Dans une telle émulsion, la phase huileuse sera constituée de l'huile peroxydée ou des huiles peroxydées et de l'huile de foie de morue et représentera avantageusement de 1 à 25 % en masse de ladite composition.

Lorsque la composition est sous forme d'un gel, il pourra s'agir soit d'un gel huileux, soit d'un gel mixte.

L'huile végétale peroxydée ou le mélange d'huiles végétales peroxydées et l'huile de foie de morue constitueront l'huile du gel huileux. Ce gel contiendra en outre un agent permettant d'obtenir la viscosité recherchée pour une bonne application topique du gel. Cet agent de viscosité sera de préférence de la silice colloïdale. Il pourra éventuellement contenir un ou plusieurs conservateurs et/ou un parfum ou un arôme, choisi en fonction du type d'application recherchée. Il pourra également contenir, de façon générale, tout adjuvant de formulation classiquement utilisé dans le domaine.

Bien entendu, le parfum ou l'arôme sera choisi en fonction du type d'application visée.

La composition pourra également se trouver sous la forme d'un gel mixte.

Un tel gel sera avantageusement obtenu à partir d'une huile végétale peroxydée ou d'un mélange d'huiles végétales peroxydées, d'huile de foie de morue, d'eau et d'un agent gélifiant, de préférence un agent gélifiant de type hydrophile. Ce gel mixte contiendra avantageusement de 1 à 25 % en poids de produits de type huileux (huiles peroxydées et huile de foie de morue).

Le gel mixte pourra en outre contenir éventuellement au moins un agent choisi parmi les conservateurs, les parfums ou les arômes et les agents émulsionnants.

Comme exposé précédemment, la composition de l'invention présente l'avantage de constituer un dispositif médical.

Plus précisément, la composition de l'invention, lorsqu'elle est appliquée sur la peau permet de former sur la peau un film protecteur qui permet le soin de la peau lorsqu'elle est irritée et présente des rougeurs ou des lésions superficielles. Ce film présente également l'avantage d'aider à une action de massage de la peau en favorisant la glisse des doigts sur la peau.

Ainsi, la composition de l'invention pourra de façon avantageuse être utilisée en tant que dispositif médical dans toutes les actions où l'on cherche à déposer un film protecteur sur la peau et/ou à favoriser une action de massage pour faciliter la régénération cutanée.

Elle pourra en particulier être utilisée pour le soin des peaux irritées et/ou présentant des rougeurs et/ou présentant des lésions superficielles, pour tous les soins de la peau lésée ou enflammée : petites plaies, écorchures, irritations, abrasions , brûlures...

Un exemple d'application où la composition de l'invention s'avère particulièrement avantageuse est celui de la prévention et/ou du soin des escarres. La composition, très riche en composés lipidiques, a une très grande affinité constitutionnelle avec la peau Elle la protège , restaure les parties lésées. Elle forme de plus un film lipidique qui agit comme un pansement à l'abri duquel la multiplication cellulaire est accélérée. Les acides gras essentiels apportés par la composition sont des éléments indispensables aux mécanismes de synthèse biologique de la régénération tissulaire.

Dans toutes les applications visées, on a pu observer, au cours de tests faits sur des volontaires, une diminution de l'inflammation, un rapide soulagement avec disparition de la sensation de brûlure ou démangeaison et une accélération du processus de cicatrisation.

### EXEMPLE

Dans l'exemple qui suit, les proportions données sont exprimées en pourcentage en poids.

La composition contient :

| | % |
|---|---|
| Huile de maïs peroxydée présentant un taux de peroxydation compris entre 50 et 300 milliéquivalents/kg | 91.1 % |
| Huile de foie de morue winterisée | 5.00 % |
| Phenoxyéthanol- parabens | 0.9 % |
| Parfum | 3.0 % |

Cette composition est conditionnée dans des flacons en verre munis d'une pompe doseuse atmosphérique (sans gaz pulseur) délivrant une dose adéquate et toujours identique de produit.

Cette composition a été utilisée dans la prévention des escarres.

On a pu observer une nette amélioration de la rougeur d'appui chez des patients allongés et menacés d'apparition d'escarres.

Un massage léger réalisé 2 fois par jour, avec cette composition, par effleurage de la zone de peau concernée (du bout des doigts), a permis de prévenir la formation d'escarres et de faciliter le soin et le massage.

Ces tests ont été réalisés sur une vingtaine de volontaires hospitalisés dans le cadre d'un long séjour.

## Revendications

1. Composition formulée pour une application topique sur la peau, **caractérisée en ce qu'**elle contient, à titre de constituants essentiels, une huile peroxydée d'origine végétale ou un mélange d'huiles peroxydées d'origine végétale présentant un taux de peroxydation compris entre 30 et 500 milliéquivalents par kg et de l'huile de foie de morue, pour son utilisation dans le soin et/ou la prévention des escarres.

2. Composition selon la revendication 1 pour son utilisation selon la revendication 1, **caractérisée en ce que** ladite huile peroxydée ou ledit mélange d'huiles peroxydées présente un taux de peroxydation compris entre 50 et 300, de préférence entre 50 et 150, milliéquivalents par kg de ladite huile peroxydée ou dudit mélange d'huiles peroxydées.

3. Composition selon l'une des revendications 1 ou 2 pour son utilisation selon la revendication 1, **caractérisée en ce que** ladite huile peroxydée ou ledit mélange d'huiles peroxydées comprend, à titre de constituants majoritaires, des triglycérides partiellement oxydés répondant à la formule générale : 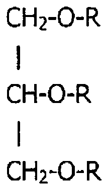 dans laquelle les radicaux R sont des acides insaturés en C18 partiellement peroxydés.

4. Composition selon une des revendications 1 à 3 pour son utilisation selon la revendication 1, **caractérisée en ce que** l'huile naturelle est choisie dans le groupe constitué de l'huile d'amande douce, de l'huile de noisette, de l'huile d'arachide, de l'huile de maïs, de l'huile de pépin de raisin, de l'huile de sésame et de l'huile de carthame et de leurs mélanges.

5. Composition selon l'une des revendications 1 à 4 pour son utilisation selon la revendication 1, **caractérisée en ce qu'**elle contient de 0,5 à 20%, de préférence de 1 a 10% en poids d'huile de foie de morue.

6. Composition selon l'une des revendications 1 à 5 pour son utilisation selon la revendication 1, **caractérisée en ce qu'**elle est sous la forme d'une émulsion de type huile dans eau, la phase huileuse de ladite émulsion étant constituée de ladite huile peroxydée ou dudit mélange d'huiles peroxydées et de l'huile de foie de morue et représentant de 1 à 25% en masse de ladite composition.

7. Composition selon l'une des revendications 1 à 5 pour son utilisation selon la revendication 1, **caractérisée en ce qu'**elle est sous la forme d'un gel huileux.

8. Composition selon la revendication 7 pour son utilisation selon la revendication 1, **caractérisée en ce que** ledit gel huileux est constitué de l'huile peroxydée ou dudit mélange d'huiles peroxydées, d'huile de foie de morue, d'un agent gélifiant, en particulier de silice colloïdale, et éventuellement de conservateurs et/ou de parfum ou d'arôme et/ou adjuvants destinés à améliorer la galénique ou la protection de la composition.

9. Composition selon l'une des revendications 1 à 5 pour son utilisation selon la revendication 1, **caractérisée en ce qu'**elle est sous la forme d'un gel mixte obtenu à partir, l'huile peroxydée ou dudit mélange d'huiles peroxydées, d'huile de foie de morue, d'eau et d'un agent gélifiant de type hydrophile, ledit gel mixte contenant de 1 à 25% en poids de l'huile peroxydée ou dudit mélange d'huiles peroxydées, et d'huile de foie de morue, et contenant éventuellement au moins un agent choisi parmi les conservateurs, les parfums ou arômes et les agents émulsionnants.

10. Dispositif médical, **caractérisé en ce qu'**il est constitué d'une composition selon l'une des revendications 1 à 9.

11. Composition selon l'une des revendications 1 à 9, ou dispositif médical selon la revendication 10, **caractérisé en ce qu'**il est destiné à former un film protecteur sur la peau, notamment pour le soin des peaux irritées et/ou présentant des rougeurs et/ou présentant des lésions superficielles et/ou à être utilisé comme adjuvant dans des opérations de massage.

## Patentansprüche

1. Zusammensetzung, die für eine topische Anwendung auf der Haut formuliert ist, **dadurch gekennzeichnet, daß** sie als wesentliche Bestandteile ein peroxidiertes Öl pflanzlicher Herkunft oder eine Mischung aus peroxidierten Ölen pflanzlicher Herkunft, mit einem Peroxidationsgrad zwischen 30 und 500 Milliäquivalenten pro kg, sowie Lebertran, für ihre Verwendung bei der Pflege und/oder der Vorbeugung von Dekubitus enthält.

2. Zusammensetzung nach Anspruch 1, für ihre Verwendung nach Anspruch 1, **dadurch gekennzeichnet, daß** das peroxidierte Öl oder die Mischung aus peroxidierten Ölen einen Peroxidationsgrad zwischen 50 und 300, vorzugsweise zwischen 50 und 150 Milliäquivalenten pro kg des peroxidierten Öls oder der Mischung aus peroxidierten Ölen aufweist.

3. Zusammensetzung nach einem der Ansprüche 1 oder 2, für ihre Verwendung nach Anspruch 1, **dadurch gekennzeichnet, daß** das peroxidierte Öl oder die Mischung aus peroxidierten Ölen als Hauptbestandteile teilweise oxidierte Triglyceride umfaßt, welche der folgenden allgemeinen Formel entsprechen: 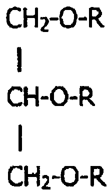 worin die Radikale R teilweise peroxidierte ungesättigte C18-Säuren sind.

4. Zusammensetzung nach einem der Ansprüche 1 bis 3, für ihre Verwendung nach Anspruch 1, **dadurch gekennzeichnet, daß** das natürliche Öl aus der Gruppe bestehend aus Süßmandelöl, Haselnußöl, Erdnußöl, Maisöl, Traubenkernöl, Sesamöl und Distelöl sowie deren Mischungen ausgewählt ist.

5. Zusammensetzung nach einem der Ansprüche 1 bis 4, für ihre Verwendung nach Anspruch 1, **dadurch gekennzeichnet, daß** sie 0,5 bis 20 Gew.-%, vorzugsweise 1 bis 10 Gew.-% Lebertran enthält.

6. Zusammensetzung nach einem der Ansprüche 1 bis 5, für ihre Verwendung nach Anspruch 1, **dadurch gekennzeichnet, daß** sie in Form einer Emulsion vom Typ Öl-in-Wasser vorliegt, wobei die Ölphase der Emulsion von dem peroxidierten Öl oder der Mischung aus peroxidierten Ölen und dem Lebertran gebildet ist und 1 bis 25 Masseprozent der Zusammensetzung ausmacht.

7. Zusammensetzung nach einem der Ansprüche 1 bis 5, für ihre Verwendung nach Anspruch 1, **dadurch gekennzeichnet, daß** sie in Form eines öliges Gels vorliegt.

8. Zusammensetzung nach Anspruch 7, für ihre Verwendung nach Anspruch 1, **dadurch gekennzeichnet, daß** das ölige Gel von dem peroxidierten Öl oder der Mischung aus peroxidierten Ölen, von Lebertran, einem Geliermittel, insbesondere kolloidalem Siliziumdioxid, sowie eventuell Konservierungsmitteln und/der Duftstoff oder Aroma und/oder Zusatzmitteln, die dazu bestimmt sind, die Galenik oder den Schutz der Zusammensetzung zu verbessern, gebildet ist.

9. Zusammensetzung nach einem der Ansprüche 1 bis 5, für ihre Verwendung nach Anspruch 1, **dadurch gekennzeichnet, daß** sie in Form eines Mischgels vorliegt, das aus dem peroxidierten Öl oder der Mischung aus peroxidierten Ölen, Lebertran, Wasser und einem Geliermittel vom hydrophilen Typ erhalten wird, wobei das Mischgel 1 bis 25 Gew.-% des peroxidierten Öls oder der Mischung aus peroxidierten Ölen und Lebertran enthält sowie eventuell wenigstens ein Mittel, das aus den Konservierungsmitteln, den Duftstoffen oder Aromen und den Emulgatoren ausgewählt ist, enthält.

10. Medizinische Vorrichtung, **dadurch gekennzeichnet, daß** sie von einer Zusammensetzung nach einem der Ansprüche 1 bis 9 gebildet ist.

11. Zusammensetzung nach einem der Ansprüche 1 bis 9, oder medizinische Vorrichtung nach Anspruch 10, **dadurch gekennzeichnet, daß** sie dazu bestimmt ist, einen Schutzfilm auf der Haut zu bilden, insbesondere für die Pflege gereizter und/oder Rötungen aufweisender und/oder oberflächliche Verletzungen aufweisender Haut, und/oder als Zusatzmittel bei Massagen verwendet zu werden.

## Claims

1. A composition formulated for topical application to the skin, **characterized in that** it contains, as essential ingredients, cod liver oil and a peroxidized oil of vegetable origin or a mixture of peroxidized oils of vegetable origin presenting a level of peroxidation comprised between 30 mEq/kg and 500 mEq/kg and cod liver oil, for its use is for the care and/or prevention of bed sores.

2. A composition according to claim 1, for its use according to claim 1, **characterized in that** said peroxidized oil or said mixture of peroxidized oils presents a level of peroxidation comprised between 50 mEq/kg and 300 mEq/kg, preferably in between 50 mEq/kg and 150 mEq/kg, of said peroxidized oil or of said mixture of peroxidized oils.

3. A composition according to claim 1 or claim 2, for its use according to claim 1, **characterized in that** the main ingredients of said peroxidized oil or said mixture of peroxidized oils include partially oxidized triglycerides that satisfy the general formula: 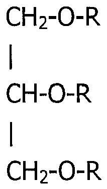 in which the radicals R are partially peroxidized C₁₈ unsaturated acids.

4. A composition according to any one of claims 1 to 3, for its use according to claim 1, **characterized in that** the natural oil is selected from the group consisting of sweet almond oil, hazelnut oil, peanut oil, corn oil, grape seed oil, sesame oil, and safflower oil, and mixtures thereof.

5. A composition according to any one of claims 1 to 4, for its use according to claim 1, **characterized in that** it contains from 0.5% to 20%, preferably from 1% to 10%, by weight of cod liver oil.

6. A composition according to any one of claims 1 to 5, for its use according to claim 1, **characterized in that** it is in the form of an oil-in-water type emulsion, the oily phase of said emulsion being constituted by cod liver oil and by said peroxidized oil or said mixture of peroxidized oils, and representing from 1% to 25% by weight of said composition.

7. A composition according to any one of claims 1 to 5, for its use according to claim 1, **characterized in that** it is in the form of an oily gel.

8. A composition according to claim 7, for its use according to claim 1, **characterized in that** said oily gel is constituted by cod liver oil, peroxidized oil or said mixture of peroxidized oils, a gelling agent, in particular colloidal silica, and possibly preservatives, and/or fragrance or aroma, and/or additives for improving its galenic form or protection of the composition.

9. A composition according to any one of claims 1 to 5, for its use according to claim 1, **characterized in that** it is in the form of a mixed gel that is obtained from cod liver oil, peroxidized oil or said mixture of peroxidized oils, water, and a hydrophilic-type gelling agent, said mixed gel containing from 1% to 25% by weight of cod liver oil and of peroxidized oil or of said mixture of peroxidized oils, and possibly containing at least one agent selected from preservatives, fragrances or aromas, and emulsifying agents.

10. A medical device **characterized in that** it is constituted by a composition according to any one of claims 1 to 9.

11. A composition according to any one of claims 1 to 9, or a medical device according to claim 10, **characterized in that** it is for forming a protective film on the skin, in particular for the care of skin that is irritated and/or that presents redness and/or that presents surface lesions, and/or it is for using as an additive in massaging operations.
